# EUROPÄISCHE PATENTSCHRIFT

(11) **EP 1 068 174 B1**
(45) Veröffentlichungstag und Bekanntmachung des Hinweises auf die Patenterteilung: **28.05.2003**
(21) Anmeldenummer: 99919140.6
(22) Anmeldetag: 25.03.1999
(51) Int. Cl.: C07C 57/07, C07C 57/05

(54) **VERFAHREN ZUR HERSTELLUNG VON ACRYLSÄURE UND ACRYLSÄUREESTERN**
METHOD FOR PRODUCING ACRYLIC ACID AND ACRYLIC ACID ESTERS
PROCEDE DE PREPARATION D'ACIDE ACRYLIQUE ET D'ESTERS D'ACIDE ACRYLIQUE

(30) Priorität: 31.03.1998 DE 19814421
(43) Veröffentlichungstag der Anmeldung: 17.01.2001
(73) Patentinhaber: BASF AKTIENGESELLSCHAFT, 67056 Ludwigshafen (DE)
(72) Erfinder: BOLZ, Gerhard, D-67227 Frankenthal (DE); NESTLER, Gerhard, D-67061 Ludwigshafen (DE); SCHRÖDER, Jürgen, D-67071 Ludwigshafen (DE)
(74) Vertreter: Isenbruck, Günter
(86) Internationale Anmeldenummer: EP9902036
(87) Internationale Veröffentlichungsnummer: WO99050222

(56) Entgegenhaltungen:
- EP-A- 0 297 788
- GB-A- 2 096 601
- US-A- 3 405 172
- US-A- 4 554 054

## Beschreibung

Die vorliegende Erfindung betrifft ein Verfahren zur Herstellung von Acrylsäure, in dem ein bei der Gasphasenoxidation zur Herstellung von Acrylsäure anfallendes gasförmiges Reaktionsgemisch, das Acrylsäure enthält, mittels einer Acrylsäure, die maximal 5 Gew.-% Wasser enthält, abgekühlt wird, und ein Acrylsäure enthaltendes gasförmiges Gemisch erhalten wird. Darüber hinaus betrifft sie ganz allgemein die Verwendung einer Acrylsäure, die maximal 5 Gew.-% Wasser enthält, zum Abkühlen eines bei der Gasphasenoxidation zur Herstellung von Acrylsäure anfallenden gasförmigen Reaktionsgemisches, sowie ein Verfahren zur Herstellung von Acrylsäureestern.

Acrylsäure bildet aufgrund ihrer sehr reaktionsfähigen Doppelbindung sowie der Säurefunktion ein wertvolles Monomeres zur Herstellung von Polymerisaten, z.B. fiir als Klebstoffe geeignete wäßrige Polymerisatdispersionen.

Unter anderem ist Acrylsäure zugänglich durch Gasphasenoxidation von Propylen und/oder Acrolein mit Sauerstoff oder Sauerstoff enthaltenden Gasen in Gegenwart von Katalysatoren bei erhöhter Temperatur, infolge der hohen Reaktionswärme vorzugsweise unter Verdünnen der Reaktionspartner mit Inertgasen und/oder Wasserdampf.

Als Katalysatoren werden dabei in der Regel oxidische Mehrkomponentensysteme, z.B. auf Basis von Molybdän-, Chrom-, Vanadium- oder Telluroxiden, eingesetzt.

Bei diesem Verfahren wird jedoch nicht reine Acrylsäure, sondern ein Gasgemisch, das neben Acrylsäure als Nebenkomponenten im wesentlichen nicht umgesetztes Acrolein und/oder Propylen, Wasserdampf, Kohlenoxide, Stickstoff, Sauerstoff, Essigsäure, Formaldehyd, Benzaldehyd, Furfurale und Maleinsäureanhydrid enthält, erhalten, von welchem die Acrylsäure anschließend abgetrennt werden muß.

Die Isolierung der Acrylsäure aus dem gasförmigen Reaktionsgemisch erfolgt in der Regel durch Gegenstromabsorption z.B. mit einem hochsiedenden Lösungsmittel bzw. Lösungsmittelgemisch und mehreren anschließenden destillativen Aufarbeitungsschritten, wie dies z.B. in der DE-A 21 36 396 und der DE-A 43 08087 beschrieben wird. Gemäß der EP-B 0 009 545, US 5 154 800, DE-A 34 29 391 sowie der DE-A 21 21 123 wird zunächst mit Wasser/wäßriger Acrylsäure im Gegenstrom absorbiert und anschließend extraktiv oder azeotrop destilliert.

Aus der US 3,405,172 ist ein Verfahren zur Herstellung von Acrylsäure bekannt wonach das gasförmige Reaktionsgemisch mittels flüssiger Acrylsäure und wässriger Acrylsäurelösung gequenscht und dabei auf eine Temperatur im Bereich von ca. 0°C bis ca. 100°C abgekühlt wird, wobei die Acrylsäure aus dem gasförmigen Reaktionsgemisch kondensiert.

Nachteilig bei diesen Verfahren ist es, daß sie in der Regel technisch und energetisch aufwendig sind und daß zur Absorption bzw. Extraktion ein zusätzliches organisches Lösungsmittel/Lösungsmittelgemisch notwendig ist, das in einem eigenen Destillationsschritt wieder abgetrennt und gegebenenfalls vor der Wiederverwendung gereinigt werden muß.

Ein weiterer Nachteil dieser Verfahren besteht darin, daß die bei der Acrylsäure-Herstellung anfallende Essigsäure (Gehalt: 0,5 bis 10 Gew.-%) bezogen auf die Menge der Acrylsäure) in einer aufwendigen Destillationsstufe abgetrennt werden muß. Aufgrund der geringen Siedepunktsunterschiede und der hohen Polymerisationsneigung der Acrylsäure erfordert dies i.d.R. mehrere Destillationsschritte, wie sich u.a. der US 3 844 903 entnehmen läßt, und verursacht beträchtliche Verluste an Acrylsäure (vgl. EP-A 398 226).

Im Hinblick auf die bekannte Tatsache, daß Acrylverbindungen eine große Neigung zur Polymerisation besitzen, sind ganz allgemein Verfahren mit mehrstufiger destillativer Aufarbeitung nachteilig, da sie die Polymerisationstendenz der Acrylsäure noch verstärken.

Eine Aufgabe der vorliegenden Erfindung besteht nunmehr darin, ein einfaches Verfahren zur Gewinnung von Acrylsäure zur Verfügung zu stellen, das zum einen kein zusätzliches Lösungs-/Absorptions- bzw. Extraktionsmittel benötigt und das darüber hinaus energetisch günstig durchführbar ist.

Auch die Herstellung von Acrylsäureestern durch säurekatalysierte Veresterung von Acrylsäure mit einem oder mehreren Alkanolen ist aus dem Stand der Technik bekannt. Bzgl. derartiger Veresterungsreaktionen ist ganz allgemein bekannt, daß es sich um Gleichgewichtsreaktionen handelt und von daher die Anwesenheit von Wasser im Reaktionsgleichgewicht wirtschaftliche Umsätze verhindert. Demgemäß wird i.d.R. weitgehend wasserfreie Acrylsäure eingesetzt und das bei der Veresterung entstehende Reaktionswasser, ggf. mit Hilfe eines Schleppmittels destillativ entfernt.

Wie bereits eingangs erwähnt, entstehen bei der Herstellung von Acrylsäure durch Oxidation ausgehend von den entsprechenden C₃-/C₄ Vorläufern noch beträchtliche Mengen an Essigsäure (0,5 bis 10 Gew.-%) und Maleinsäure/Maleinsäureanhydrid (0,1 bis 1 Gew.- %). Aufgrund der zum Teil geringen Siedepunktsunterschiede und der hohen Polymerisationsneigung der Acrylsäure bei thermischer Belastung, ist eine destillative Abtrennung der o.g. Nebenprodukte schwierig und aufwendig (US 3 844 903, DE-A 2 164 767).

Da bei der Veresterung von essigsäurehaltiger Acrylsäure mit Alkanolen auch die Essigsäure verestert wird, bedingt die Bildung des Essigesters einen zusätzlichen Trennaufwand und einen Verlust an Alkanol. Dabei ist ferner zu beachten, daß die destillative Abtrennung des Essigsäurealkylesters aus dem Veresterungsgemisch; vor allem die Trennung von nicht umgesetztem Alkanol, wegen der Ausbildung von binären Azeotropen, behindert wird.

Im Falle von z.B. Butanol siedet das Butanol-Butylacetat-Azeotrop bei 115,8 °C (57 % Butanol), wobei Butanol bei 117,4 °C und Butylacetat bei 125,6 °C sieden.

Da die Essigsäureester relativ leicht flüchtig und nicht polymerisierbar sind, werden bei der Herstellung von Polymerisaten i.d.R. hochreine Acrylsäureester, d.h. Acrylsäureester, die möglichst, d.h. im Wesentlichen, frei von Essigsäureestern sind, benötigt. Der beispielsweise in einer Anstrichdispersion oder in einem Klebstoff verbleibende Essigsäureester würde nämlich u.a. eine starke Geruchsbelästigung hervorrufen. Eine aufwendige Entfernung (Desodorierung) des Essigsäureesters wäre notwendig.

Darüber hinaus verbraucht die in der Acrylsäure enthaltene Maleinsäure Alkanol, da bei der Veresterung auch die entsprechenden Maleinsäurealkylester gebildet werden.

Wie sich aus obigem ergibt, besteht bei der Acrylsäureester-Herstellung prinzipiell das Problem eines zu hohen Verbrauchs an Alkanolen, was sowohl wirtschaftlich als auch ökologisch nachteilig ist.

Es wurden daher in der Vergangenheit bereits verschiedene Versuche unternommen, die Probleme, die die bei der Synthese von Acrylsäure durch Gasphasenoxidation anfallenden Nebenprodukte, wie z.B. Essigsäure und Wasser, bei der Veresterung mit Alkanolen verursachen, zu losen.

So beschreibt die DE-A 2 035 228 die Veresterung von wäßriger Acrylsäure (Gehalt an Wasser: mindestens 30 %) in Gegenwart von sauren Katalysatoren, wie z.B. Schwefel-, Sulfon-, Phosphorsäure und einem organischem Lösungsmittelgemisch.

Dieses Verfahren ist insbesondere dahingehend nachteilig, daß bedingt durch die große Menge an Wasser, die zu einer Verringerung der Katalysatorkonzentration führt, eine große Menge an Katalysator verwendet werden muß. Um brauchbare Umsätze bzw. Veresterungsgeschwindigkeiten zu erreichen, müssen darüber hinaus gemäß dieser Druckschrift spezielle Lösungsmittelgemische, bestehend aus einem aromatischen und einem aliphatischen Kohlenwasserstoff verwendet werden. Eine weitere Bedingung zur erfolgreichen Durchführung dieses Verfahrens liegt darin, daß das Lösungsmittelgemisch außerdem deutlich höher sieden muß als der Acrylsäureester.

Die EP-A 0 398 226 schlägt vor, eine partielle Kondensation der Reaktionsgase der Propylenoxidation durchzuführen und die dabei anfallende Acrylsäure (enriched acylic acid) direkt zu verestern und die im Reaktionsgas verbleibende Acrylsäure auf herkömmliche Weise mit Wasser auszuwaschen und destillativ abzutrennen. Dieses Verfahren der zweistufigen Acrylsäurekondensation ist sehr aufwendig und liefert keine essigsäurefreie bzw. -arme Acrylsäure. Wie die dort wiedergegebenen Beispiele zeigen, enthalten die erhaltenen Veresterungsgemische noch 1,8 bis 2,5 Gew.- % Essigsäureester.

Gemäß der DE-A 1 668 362 wird das acrylsäurehaltige Reaktionsgas der Propylenoxidation mit den bei der Veresterung anfallenden Hochsiedergemischen, die im wesentlichen aus Maleinsäureester, Polyacrylsäure und Polyacrylsäureestern bestehen, behandelt und die dabei anfallende Acrylsäurelösung destillativ von den Leichtsiedern befreit. Die so erhaltene acrylsäurehaltige Sumpflösung wird mit Alkanolen in Gegenwart von sauren Kationenaustauschern verestert.

Nachteilig bei diesem Verfahren ist, daß die Acrylsäureaufarbeitung mit der Esterherstellung gekoppelt ist, wobei man die Möglichkeit zur Herstellung verschiedener Ester verliert. Darüber hinaus werden bedingt durch die Bildung von hochsiedenden Estern der Polyacrylsäure, deren Überschuß entsorgt werden muß, beträchtliche Alkanolverluste verursacht.

Die JA 7 014 529-R beschreibt die Herstellung von Butylacrylat aus wäßriger Acrylsäure. Gemäß des darin beschriebenen Verfahrens wird Acrylsäure mit einem Butylacrylat-Butanol-Gemisch aus der wäßrigen Lösung extrahiert und anschließend das Extrakt, das ca. 18 Gew.-% Acrylsäure, ca. 1 Gew.-% Essigsäure und ca. 11 Gew.-% Wasser enthält, mit einem Alkanol verestert.

Nachteilig ist bei diesem Verfahren vor allem, daß große Wasser- und Essigsäuremengen in die Veresterung eingeschleust werden, was die Veresterung in bekannter Weise negativ beeinflußt und die Aufarbeitung des Veresterungsgemisches auf bekannte Weise stört und Alkanolverluste verursacht.

Die FR-B 1 452 566 betrifft die Extraktion von Acrylsäure aus einer wäßrigen Lösung mit Acetophenon oder Tributylphosphat und die Veresterung dieser Acrylsäure mit überschüssigem Alkohol in Anwesenheit eines Extraktionsmittels. Die Ausbeute an Acrylsäureester gemäß dieses Verfahrens beträgt jedoch weniger als 80 %, bezogen auf die in der Ausgangslösung vorhandene Acrylsäure.

Aufgrund der negativen Auswirkung, die die Anwesenheit nennenswerter Mengen an Wasser, Essigsäure und/oder Maleinsäure/Maleinsäureanhydrid auf die Herstellung von Acrylsäureestern besitzt, wird i.d.R. wasserfreie und gereinigte Acrylsäure, die lediglich Spuren an Essigsäure und Maleinsäure/Maleinsäureanhydrid enthält, zur Herstellung von Acrylsäureestern verwendet.

Eine weitere Aufgabe der vorliegenden Erfindung besteht nunmehr darin, ein technisch einfaches und wirtschaftliches Verfahren zur Herstellung von Acrylsäureestern zur Verfügung zu stellen, bei dem trotz Anwesenheit von Essigsäure und Maleinsäure/Maleinsäureanhydrid in der eingesetzten Acrylsäure die Alkanolverluste gering sind.

Demgemäß betrifft die vorliegende Erfindung ein Verfahren zur Herstellung von Acrylsäure, das folgende Stufe A umfaßt:
- A:: Abkühlen eines bei der Gasphasenoxidation zur Herstellung von Acrylsäure anfallenden gasförmigen Reaktionsgemisches, das Acrylsäure enthält, mittels einer Acrylsäure, die maximal 5 Gew.- % Wasser enthält, auf eine Temperatur von 100 bis 190°C, wobei ein Acrylsäure enthaltendes, gasförmiges Gemisch erhalten wird.

Der Begriff "gasförmiges Reaktionsgemisch, das Acrylsäure enthält" umfaßt erfindungsgemäß alle Reaktionsgemische, die bei der Gasphasenoxidation zur Herstellung von Acrylsäure anfallen.

Hier erfolgt die Herstellung von Acrylsäure auf bekannte Weise an oxidischen Mehrkomponentenkatalysatoren bei ungefähr 200 bis 400 °C. Obwohl prinzipiell alle bekannten Reaktortypen verwendet werden, setzt man bevorzugt Rohrbündelwärmeaustauscher ein, die mit dem Oxidationskatalysator gefüllt sind. Der Grund hierfür liegt darin, daß der überwiegende Teil der bei der Oxidation freiwerdenden Wärme durch Konvektion und Strahlung an die gekühlten Rohrwände abgeführt werden kann.

Geht man von Propylen/Acrolein als Edukte zur Herstellung von Acrylsäure aus, handelt es sich um ein gasförmiges Reaktionsgemisch, das mit einer Temperatur von ungefähr 200 bis 300 °C aus der Gasphasenoxidation anfällt und ungefähr 1 bis ungefähr 30 Gew.-% Acrylsäure enthält und als Nebenprodukte nicht umgesetztes Propylen (ungefähr 0,05 bis ungefähr 1 Gew.-%), Acrolein (ungefähr 0,001 bis ungefähr 2 Gew.-%), Propan (ungefähr 0,01 bis ungefähr 2 Gew.-%), Wasserdampf (ungefähr 1 bis ungefähr 30 Gew.-%), Kohlenoxide (ungefähr 0,05 bis ungefähr 15 Gew.-%), Stickstoff (0 bis ungefähr 90 Gew.-%), Sauerstoff (ungefähr 0,05 bis ungefähr 10 Gew.-%), Essigsäure (ungefähr 0,05 bis ungefähr 2 Gew.-%), Propionsäure (ungefähr 0,01 bis ungefähr 2 Gew.-%), Aldehyde (ungefähr 0,05 bis ungefähr 3 Gew.-%) und Maleinsäureanhydrid (ungefähr 0,01 bis ungefähr 0,5 Gew.-%) umfaßt.

Dieses gasförmige Reaktionsgemisch wird mittels Acrylsäure, die maximal 5 Gew.-% Wasser, vorzugsweise maximal 3 Gew.-% Wasser, enthält, in der Regel auf eine Temperatur von ungefähr 100 bis ungefähr 190°C, vorzugsweise ungefähr 120 bis ungefähr 180°C und insbesondere ungefähr 130 bis ungefähr 160°C abgekühlt, wobei ein wiederum gasförmiges, Acrylsäure enthaltendes Gemisch erhalten wird.

Der Begriff "Acrylsäure, die maximal 5 Gew.-% Wasser enthält" umfaßt sowohl Rein-Acrylsäure, als auch nach einem beliebigen Verfahren erhaltene Roh-Acrylsäure, die die oben genannte Bedingung erfüllt. Vorzugsweise wird jedoch die gemäß Stufe B, wie im folgenden beschrieben, erhaltene Roh-Acrylsäure zur Abkühlung verwendet.

Vorzugsweise wird die zur Abkühlung verwendete Acrylsäure, die maximal 5 Gew.-% Wasser enthält, im Kreis und insbesondere zwecks Wärmeabfluß über einen herkömmlichen Wärmeaustauscher gefahren.

Vorzugsweise wird diese Acrylsäure mit Stabilisatoren, wie z.B. Phenothiazin, Hydrochinon, einer phenolischen Verbindung oder ein Gemisch aus zwei oder mehr davon versetzt. Vorzugsweise werden Phenothiazin, Hydrochinon oder eine Gemisch aus Phenothiazin und einer N-O-Verbindung, wie z.B. p-Nitrosophenol, p-Nitrosodiethylanilin oder einem Tetramethyl-piperidin-1-oxyl als Stabilisatoren eingesetzt.

Als Abkühlvorrichtung können alle aus dem Stand der Technik für diesen Zweck bekannten Vorrichtungen eingesetzt werden, wobei vorzugsweise Venturi-Wäscher oder Sprühkühler (Quench) und insbesondere Sprühkühler verwendet werden.

Das so erhaltene, Acrylsäure enthaltende gasförmige Gemisch wird vorzugsweise in einer Stufe B aufgetrennt, wobei eine Leichtsiederfraktion und eine Roh-Acrylsäure (als Sumpfprodukt) erhalten wird. Insbesondere wird die Auftrennung gemäß Stufe B in einer Destillationskolonne durchgeführt.

Dabei wird das abgekühlte, Acrylsäure enthaltende gasförmige Gemisch in den unteren Teil der Destillationskolonne geleitet und die Destillation so durchgeführt, daß die gasförmigen Bestandteile und die Leichtsieder, d.h. hauptsächlich Essigsäure und Wasser über den Kopf der Kolonne abgetrennt werden, während die enthaltene Acrylsäure als Roh-Acrylsäure im Sumpf der Kolonne anfällt. Die so erhaltene Roh-Acrylsäure enthält im allgemeinen ungefähr 0,1 bis ungefähr 2 Gew.-% Essigsäure, ungefähr 0,1 bis ungefähr 1 Gew.-% Maleinsäure/Maleinsäureanhydrid und ungefähr 0,5 bis ungefähr 5 Gew.-% Wasser. Darüber hinaus sind im Sumpf auch weitere Hochsieder, wie z.B. Oligo-Acrylsäure, enthalten.

Darüber hinaus betrifft die vorliegende Erfindung ein Verfahren zur Herstellung eines Acrylsäureesters oder eines Gemisches aus zwei oder mehr davon, das eine Stufe A, wie hierin definiert, eine Stufe B, wie hierin definiert, sowie eine weitere Stufe C umfaßt.
- C:: Veresterung der in Stufe B erhaltenen Roh-Acrylsäure mittels einem oder mehreren Alkanolen, wobei ein Veresterungsgemisch umfassend einen oder mehrere Acrylsäureester, einen oder mehrere Essigsäureester, einen oder mehrere Maleinsäureester und oligomere und polymere Acrylsäureester erhalten wird.

Ferner betrifft sie ein Verfahren, das zusätzlich zu den Stufen A bis C eine weitere Stufe D umfaßt:
- D:: Auftrennung des Veresterungsgemisches unter Erhalt eines oder mehrerer Acrylsäureester, sowie eines Auftrennungsgemisches, das einen oder mehrere Essigsäureester umfaßt, und eines Sumpfes, der einen oder mehrere Maleinsäureester und oligomere und polymere Acrylsäureester enthält.

Vorzugsweise werden das Auftrennungsgemisch und/oder der Sumpf anschließend verseift, wobei bei der Verseifung des Auftrennungsgemischs ein erstes Verseifungsgemisch, das ein oder mehrere Alkanole und Essigsäuresalze umfaßt, bei der Verseifung des Sumpfes ein zweites Verseifungsgemisch, das ein oder mehrere Alkanole und Maleinsäuresalze, Acrylsäuresalze, Hydroxypropionsäuresalze und polymere Acrylsäuresalze enthält, und bei der Verseifung des Auftrennungsgemischs und des Sumpfes eine Mischung aus erstem Verseifungsgemisch und zweitem Verseifungsgemisch erhalten wird (Stufe E).

Im folgenden wird zunächst das erfmdungsgemäße Verfahren zur Herstellung von Acrylsäure, das die hierin definierte Stufe A sowie wahlweise eine weitere Stufe B umfaßt, und anschließend das erfindungsgemäße Verfahren zur Herstellung eines Acrylsäureesters, das zusätzlich die Stufen C und ggf. D sowie ggf. E urnfaßt, beschrieben.

Die für das erfindungsgemäße Verfahren einsetzbaren Destillationskolonnen (Kolonnen) unterliegen keiner besonderen Beschränkung. Grundsätzlich eignen sich alle Kolonnen mit trennwirksamen Einbauten.

Als Kolonneneinbauten kommen alle gängigen Einbauten in Betracht, insbesondere Böden, Packungen und/oder Füllkörper. Von den Böden sind Glockenböden, Siebböden, Ventilböden und/oder Dual-Flow-Böden bevorzugt. Die Kolonne umfaßt wenigstens eine Kühlvorrichtung. Hierfür eignen sich alle Wärmeüberträger oder Wärmetauscher, bei denen die bei der Kondensation frei werdende Wärme indirekt (extern) abgeführt wird. Hierfür können alle gängigen Apparate eingesetzt werden, wobei Rohrbündelwärmetauscher, Plattenwärmetauscher und Luftkühler bevorzugt sind. Geeignete Kühlmedien sind bei einem Luftkühler entsprechend Luft und bei anderen Kühlvorrichtungen Kühlflüssigkeiten, insbesondere Wasser. Ist nur eine Kühlvorrichtung vorgesehen, so wird diese am Kopf der Kolonne eingebaut, in dem die Leichtsiederfraktion auskondensiert wird.

Der Fachmann kann in Abhängigkeit von der gewünschten Reinheit der kondensierten Fraktionen und damit der Komponenten die Anzahl der erforderlichen Kühlvorrichtungen leicht bestimmen, wobei die Reinheit der kondensierten Komponenten im wesentlichen durch die installierte Trennleistung der Kolonne, d.h. die Kolonnenhöhe und die über das zu kondensierende gasförmige Gemisch aus Stufe A eingetragene Energie bestimmt wird.

Zweckmäßigerweise werden bei Vorhandensein mehrerer Kühlvorrichtungen diese in verschiedenen Abschnitten der Kolonne eingebaut. Der in der Kolonne vorliegende Druck hängt von der Menge an nicht kondensierbaren Komponenten ab und beträgt vorzugsweise 0,5 - 5 bar Absolutdruck, insbesondere 0,8 - 3 bar Absolutdruck.

Die Temperatur im Bereich der Auftrennvorrichtung, in dem die Leichtsieder, d.h. im wesentlichen typischerweise Aldehyde, Essigsäure und Wasser, abgetrennt werden, beträgt ungefähr 25 bis ungefähr 50 °C, vorzugsweise ungefähr 30 bis ungefähr 40 °C. Die Temperatur im Sumpf, in dem die Roh-Acrylsäure erhalten wird, liegt im allgemeinen bei ungefähr 95 bis ungefähr 130 und insbesondere bei ungefähr 100 bis 120 °C.

Die genauen Betriebsbedingungen für die Kolonne, wie Temperatur- und Druckführung, Schaltung und Anordnung der Kühlvorrichtung(en), Wahl der Kolonnenhöhe und des Kolonnendurchmessers, Anzahl und Abstand der trennwirksamen Einbauten/Böden in der Kolonne oder Art der trennwirksamen Kolonneneinbauten, können vom Fachmann im Rahmen fachüblicher Versuche in Abhängigkeit von der Trennaufgabe ermittelt werden.

Dabei wird die Stufe B erfindungsgemäß so durchgeführt, daß der Hauptteil, in der Regel mehr als 90 Gew.-% der Acrylsäure des Acrylsäure enthaltenden gasförmigen Gemischs im Sumpf der Kolonne anfällt.

Während des Auftrennens wird zur Stabilisierung ein Polymerisationsinhibitor, wie z.B. Phenothiazin, eine phenolische Verbindung, eine N-O-Verbindung oder ein Gemisch aus zwei oder mehr davon, vorzugsweise Phenothiazin oder Hydrochinon, ein Gemisch aus Phenothiazin und Hydrochinon, Hydrochinonmonomethylether, p-Nitrosophenol, Nitrosodiethylanilin oder Tetramethylpiperidin-1-oxylen, wie sie in der DE-A 16 18 141 beschrieben sind, zugegeben.

Die nach der Auftrennung erhaltenen Leichtsieder werden nach dem Ausschleusen aus der Auftrennvorrichtung ganz oder teilweise, gegebenenfalls unter Zugabe eines Polymerisationsinhibitors als Rücklauf wieder in den oberen Teil der Auflrennvorrichtung zurückgeführt, um dort die Kondensation der im Acrylsäure enthaltenden gasförmigen Gemisch enthaltenen Leichtsieder zu erleichtern.

Die erhaltene Roh-Acrylsäure wird ganz oder teilweise der Kristallisation bzw. Destillation nach einem aus dem Stand der Technik bekannten Verfahren unterworfen, wobei eine Rein-Acrylsäure erhalten wird. Dabei wird Mutterlauge aus der Kristallisationsstufe ganz oder teilweise und/oder ggf. ein Teil der Roh-Acrylsäure in die Kolonne zurückgeführt.

Darüber hinaus kann, wie eingangs erwähnt, die erfindungsgemäß erhaltene Roh-Acrylsäure vollständig oder teilweise, vorzugsweise teilweise, der Veresterung nach einem Verfahren gemäß des Standes der Technik, wie dies beispielsweise in der DE-A 195 47 485 und dem dort zitierten Stand der Technik beschrieben ist, zugeführt werden, wobei die DE-A 195 47 485 voll umfänglich in den Kontext der vorliegenden Anmeldung einbezogen wird.

In einer bevorzugten Ausführungsform wird das erfindungsgemäße Verfahren zur Acrylsäure-Herstellung bzw. Acrylsäureester-Herstellung wie folgt durchgeführt.

Ein Teil der in Stufe B erhaltenen Roh-Acrylsäure wird standgeregelt, entsprechend dem Acrylsäurestand bei der Abkühlung (im Quench), dem Quenchkreis zur Abkühlung der Reaktionsgase zugeführt. Diese Zuführung erfolgt typischerweise über eine Abkühlvorrichtung, vorzugsweise einen herkömmlichen Wärmeaustauscher, die dem Quench vorgeschaltet ist, in der (dem) die Roh-Acrylsäure auf eine Temperatur von im allgemeinen ungefähr 40 bis ungefähr 100 °C, vorzugsweise ungefähr 80 bis 100 °C abgekühlt wird, und zum Abkühlen des bei der Gasphasenoxidation zur Herstellung von Acrylsäure anfallenden gasförmigen Reaktionsgemisches, das Acrylsäure enthält, in Stufe A zurückgeführt wird.

Ein weiterer Teil der in Stufe B erhaltenen Roh-Acrylsäure wird, vorzugsweise über einen Wärmetauscher, in die Kolonne zurückgeführt.

Der überschüssige Teil der Roh-Acrylsäure wird direkt mit einem C₁-C₁₂-, vorzugsweise C₁-C₁₀-Alkanol, insbesondere C₄-C₈-Alkanol, verestert. Dabei wird die Veresterung nach einem Verfahren des Standes der Technik, z.B. dem Verfahren der DE-A 195 47 485, durchgeführt, wobei die Veresterungsbedingungen vom verwendeten Alkanol abhängig sind.

Als bevorzugte Alkanole sind zu nennen:
Methanol, Ethanol, iso-Propanol, n-Propanol, n-Butanol, iso-Butanol, Octanol, 2-Ethylhexanol, vorzugsweise n-Butanol, iso-Butanol und 2-Ethylhexanol.

Typische Bedingungen, unter denen die Veresterung stattfinden kann, sind wie folgt:
Verhältnis Alkanol:Acrylsäure:
1:0,7-1,2 (molar)

Katalysator:
Schwefelsäure oder eine Sulfonsäure, wie z.B. p-Toluolsulfonsäure

Katalysatormenge:
Ungefähr 0,1 - 10 Gew.-% vorzugsweise ungefähr 0,5 - 5 Gew.-%, jeweils bezogen auf die Einsatzstoffe

Stabilisator:
Phenothiazin, Hydrochinon, Hydrochinonmonomethylether, Phenylendiamin und ggf. Luft

Stabilisatormenge:
Ungefähr 100 - ungefähr 50.000 ppm, vorzugsweise ungefähr 500 - ungefähr 2.000 ppm, jeweils bezogen auf Acrylsäure.

Umsetzungstemperatur:
Ungefähr 80 - 160 °C, vorzugsweise ungefähr 90 - 130 °C

Druck während der Umsetzung:
0,5 bis 1,5 bar, vorzugsweise bei Atmosphärendruck

Umsetzungsdauer:
Ungefähr 1 bis ungefähr 10 Stunden, vorzugsweise ungefähr 1 bis 6 Stunden.

Ggf. kann ein Schleppmittel, wie z.B. Cyclohexan oder Toluol zur Entfernung des während der Veresterung entstehenden Wassers eingesetzt werden.

Die Veresterung an sich kann drucklos, mit Überdruck oder Unterdruck, sowie kontinuierlich als auch diskontinuierlich durchgeführt werden, wobei eine kontinuierliche Führung des Gesamtverfahrens, d.h. eine kontinuierliche Durchführung der hierin erläuterten Stufen A bis E bevorzugt ist.

Wenn erfindungsgemäß die in Stufe B erhaltene Roh-Acrylsäure verestert wird, erhält man ein Veresterungsgemisch, das den/die gewünschten Acrylsäureester und darüber hinaus den/die entsprechenden Essigsäureester und den/die Maleinsäureester sowie oligomere und polymere Acrylsäureester umfaßt.

Die Isolierung der Acrylsäureester erfolgt auf herkömmliche Weise. In der Regel werden dabei zunächst der Katalysator und die nicht umgesetzte Acrylsäure ausgewaschen und anschließend das Veresterungsgemisch aufgetrennt, vorzugsweise destillativ aufgetrennt.

Bei dieser Auftrennung wird einerseits der eine oder mehrere Acrylsäureester und andererseits ein Auftrennungsgemisch, das einen oder mehrere Essigsäureester umfaßt, und ein Sumpf, der einen oder mehrere Maleinsäureester und polymere und oligomere Acrylsäureester enthält, erhalten. Das Auftrennungsgemisch und/oder der Sumpf werden vorzugsweise in einer weiteren Stufe E verseift, wobei ein erstes Verseifungsgemisch, das das ein oder mehrere Alkanole und Essigsäuresalze, oder ein zweites Verseifungsgemisch, das ein oder mehrere Alkanole, Maleinsäuresalze und Acrylsäuresalze, Hydroxypropionsäuresalze sowie polymere Acrylsäuresalze enthält, oder eine Mischung aus beiden erhalten wird. Aus diesen Verseifungsgemischen kann anschließend das eine oder mehrere Alkanole wiederum abgetrennt und in die Veresterung gemäß Stufe C zurückgeführt werden.

Im einzelnen wird dabei vorzugsweise wie folgt vorgegangen:

Zunächst wird das Veresterungsgemisch destillativ aufgetrennt, wobei eine Leichtsiederfraktion, die u.a. den Essigsäureester umfaßt und ein Sumpfprodukt, das die Hauptmenge des Acrylsäureesters enthält, erhalten wird. Das Sumpfprodukt wird danach ebenfalls destillativ aufgetrennt, wobei der Acrylsäureester über Kopf erhalten wird.

Die bei der destillativen Auftrennung anfallende Leichtsiederfraktion, die hauptsächlich aus Alkanol (ungefähr 20 bis ungefähr 70 %), Essigsäureester (ungefähr 5 bis ungefähr 40 %) und Acrylsäureester (ungefähr 5 bis ungefähr 50 %) besteht, wird ggf. zusammen mit dem bei der Reindestillation des gewünschten Esters anfallende Destillationssumpf, der u.a. den Maleinsäureester, oligomere und polymere Acrylsäureester und die Inhibitoren enthält, mit einer 5 bis 40 gew.-%igen wäßrigen Alkalilauge, vorzugsweise NaOH bei Siedetemperatur ungefähr 30 min bis ungefähr 10 Stunden lang behandelt.

Die Leichtsiederfraktion kann dabei ggf. noch in einem weiteren Auftrennungs-, vorzugsweise Destillationsschritt in ein hauptsächlich aus Alkanol und Acetat bestehendes Kopfprodukt und ein Sumpfprodukt, das im wesentlichen aus Acrylsäureester besteht, aufgetrennt werden. Bei dieser Reaktionsführung wird anschließend das Kopfprodukt verseift.

Der erhaltene Acrylsäureester wird vorzugsweise der destillativen Aufarbeitung des Veresterungsgemischs zugeführt.

Die Umsetzung mit Alkalilauge (Verseifung) kann kontinuierlich oder diskontinuierlich, drucklos oder bei Über- oder Unterdruck durchgeführt werden. Vorzugsweise wird dazu ein Rühr- oder Rohrreaktor eingesetzt.

Die Abtrennung des Alkanols aus dem erhaltenen Verseifungsgemisch hängt von der Art des Alkanols, d.h. von dessen Wasserlöslichkeit, ab. In Wasser unlösliche Alkanole bilden eine zweite Phase aus und können einfach abgetrennt werden. In Wasser lösliche Alkanole werden z.B. durch Destillation bzw. durch Strippen mit Luft oder Wasserdampf abgetrennt. Vorzugsweise werden die erhaltenen Alkanole dann wiederum der Veresterung zugeführt. Die destillative Abtrennung oder das Strippen kann beispielsweise in einem beheizbaren Rührreaktor mit einer aufgesetzten Kolonne erfolgen. Der Energieeintrag kann auf herkömmliche Weise erfolgen (Doppelwand-, Rohrschlangenheizung, Umlauferhitzer usw.).

Das Strippen des Alkanols in einer Strippkolonne kann auf übliche Weise erfolgen. Beispielsweise kann die heiße (ungefähr 40 bis ungefähr 80 °C) Verseifungslösung am Kopf einer Kolonne eingespeist werden und im Gegenstrom mit Luft (ungefähr 1 bis ungefähr 20 m³/m³) oder Dampf (ungefähr 0,1 bis ungefähr 10 t/m³) gestrippt werden. Die Kondensation des Alkanols aus dem Strippgas kann mit einem herkömmlichen Kühler, z.B. einem Rohrbündel- oder Plattenwärmeaustauscher, erfolgen.

Das Alkanol kann dann wieder der Veresterung gemäß Stufe C zugeführt werden.

Das erfindungsgemäße Verfahren weist die folgenden Vorteile auf:
1. Wasserarme Roh-Acrylsäure kann technisch einfach gewonnen werden. Es wird nur eine Auftrennvorrichtung, vorzugsweise eine Destillationskolonne und kein fremder Hilfsstoff, wie beispielsweise ein Lösungs- oder Extraktionsmittel, benötigt.
2. Bedingt durch eine geringe Verschmutzung durch Polymerisat weist die verwendete Anlage eine lange Laufzeit auf.
3. Wegen der Rückgewinnung der Alkanolkomponente aus den bei der Veresterung anfallenden Essigsäure-, Maleinsäureestern sowie oligomeren und polymeren Acrylsäureestern werden Verluste an Alkanol auf ein Minimum beschränkt.

In ihrer allgemeinsten Ausführungsform betrifft die vorliegende Erfindung ferner die Verwendung von Acrylsäure mit einem Wassergehalt von nicht mehr als 5 Gew.- % zum Abkühlen eines bei der Gasphasenoxidation zur Herstellung von Acrylsäure anfallenden gasförmigen Reaktionsgemischs, das Acrylsäure enthält.

Die vorliegende Erfindung soll nunmehr anhand eines Beispiels erläutert werden.

### Beispiel

Durch zweistufige katalytische Oxidation von Propylen mit molekularem Sauerstoff wurde auf übliche Weise ein gasförmiges Reaktionsgemisch mit folgender Zusammensetzung erhalten:
9,84 Gew.-% Acrylsäure,
0,4 Gew.-% Essigsäure,
4,4 Gew.-% Wasser,
0,11 Gew.-% Acrolein,
0,21 Gew.-% Formaldehyd,
0,07 Gew.-% Maleinsäureanhydrid, sowie
Propionsäure, Furfural, Propan, Propen, Stickstoff, Sauerstoff und Kohlenoxide.

Dieses gasförmige Reaktionsgemisch wurde in einem Sprühkühler (Quench) durch Eindüsen von Roh-Acrylsäure mit einem Wassergehalt von 1,5 Gew.-% (600 l/h) auf 140 °C abgekühlt. Die Roh-Acrylsäure wurde dabei über einen Wärmeaustauscher im Kreis gefahren und eine Temperatur von 95 °C eingestellt.

Das abgekühlte, gasförmige Gemisch, das Acrylsäure enthielt, wurde über einen Tropfenabscheider (Zyklon) in den unteren Teil einer Destillationskolonne geleitet, die mit 50 Dual-Flow-Böden und einem Sprühkondensator am Kopf der Kolonne ausgerüstet war. Die Temperatur am Kopf der Destillationskolonne betrug 34 °C, die Sumpftemperatur der Destillationskolonne 118 °C.

Das im Sprühkondensator anfallende Destillat, das hauptsächlich aus Wasser und Essigsäure bestand, wurde nach Ausschleusung von 20 % desselben und Zugabe von 300 ppm Hydrochinon als Rücklauf wieder auf den obersten Kolonnenboden aufgebracht.

Die im Sumpf der Kolonne anfallende Roh-Acrylsäure wurde teilweise (430 g/h) ausgeschleust, teilweise (250 g/h) mit 1000 ppm Phenothiazin stabilisiert, auf dem Boden 16 der Kolonne zugeführt und teilweise (ungefähr 5 l/h) über einen Wärmetauscher geleitet und auf Boden 3 der Kolonne zurückgeführt.

Ein Teil der anfallenden Roh-Acrylsäure wird entsprechend dem Stand im Quench diesem standgeregelt über einen weiteren, dem Quench vorgeschalteten Wärmetauscher zugeführt.

Die ausgeschleuste Roh-Acrylsäure enthielt 96,3 Gew.- % Acrylsäure, 0,9 Gew.- % Essigsäure, 0,05 Gew.- % Propionsäure, 0,4 Gew.- % Maleinsäure, 0,01 Gew.- % Acrolein, 0,3 Gew.- % Furfural und 1,5 Gew.- % Wasser.

Eine Rührkesselkaskade bestehend aus drei Rührreaktoren mit je einem Liter Reaktionsvolumen, die mit Kolonne, Kondensator und Phasentrenngefäß ausgerüstet waren, wurden mit 500 g der aus dem Quench ausgetragenen Roh-Acrylsäure, 550 g Butanol und 13 g Schwefelsäure pro Stunde beschickt. Die Reaktionstemperatur in den Reaktoren betrug 105 °C, 118 °C und 122 °C; der Druck betrug jeweils 700 mbar. Am Kopf der Kolonne fiel ein Gemisch aus Wasser, Butanol und Butylacrylat an, das in eine wäßrige und eine organische Phase zerfiel. Die organische Phase wurde mit 300 ppm Phenothiazin versetzt und der Kolonne als Rücklauf zugeführt.

Der Reaktoraustrag (929 g/h) wurde auf 30 °C abgekühlt, die nicht umgesetzte Acrylsäure und der Katalysator mit 5%iger Natronlauge neutralisiert, mit Wasser gewaschen und anschließend in einer Siebbodenkolonne mit 60 Böden destilliert. Der Zulauf zur Kolonne erfolgte auf dem 5. Boden. Die Sumpftemperatur betrug 110 °C, die Kopftemperatur betrug 88 °C; der Druck betrug 160 mbar.

Am Kopf dieser Kolonne fielen 820 g/h Destillat an, das sich in eine organische Phase (813 g/h) und eine Wasserphase trennte. 702 g/h der organischen Phase wurden mit 300 ppm Phenothiazin versetzt und als Rücklauf wieder auf den obersten Boden der Siebbodenkolonne aufgebracht.

Die organische Phase des Destillats enthielt 8,3 Gew.-% Essigsäurebutylester, 37,7 Gew.-% Butanol und 47,7 Gew.-% Butylacrylat.

Der Sumpfablauf wurde in einer weiteren Siebbodenkolonne (30 Böden) in Butylacrylat mit einer Reinheit von 99,7 % (751 g/h) als Kopfprodukt, sowie Hochsieder, die Maleinsäuredibutylester, Inhibitoren und oligomeres/polymeres Butylacrylat enthielten, als Sumpfprodukt aufgetrennt. Bei dieser Trennung betrug die Sumpftemperatur 108 °C, die Kopftemperatur 80 °C, bei einem Druck von 100 mbar. Das Rücklaufverhältnis betrug 0,6.

In einem Rührreaktor wurde ein Gemisch aus 1.170 g der organischen Phase der Leichtsiederfraktion und 420 g Destillationssumpf mit 30%-iger Natronlauge (2.400 g) zwei Stunden lang unter Rückfluß erhitzt. Nach Beendigung der Verseifungsreaktion wurde das gebildete Butanol unter Vakuum (500 mbar) über eine Kolonne (10 Glockenböden) aus dem Reaktor destillativ abgetrennt. Das Kondensat zerfiel in eine Wasserphase und eine Butanolphase (935 g). Dabei konnten ungefähr 80 % der theoretischen Butanolmenge zurückgewonnen werden.

## Patentansprüche

1. Verfahren zur Herstellung von Acrylsäure, das folgende Stufe A umfaßt:
A: Abkühlen eines bei der Gasphasenoxidation zur Herstellung von Acrylsäure anfallenden gasförmigen Reaktionsgemisches, das Acrylsäure enthält, mittels einer Acrylsäure, die maximal 5 Gew.-% Wasser enthält, auf eine Temperatur von 100 bis 190°C, wobei ein Acrylsäure enthaltendes, gasförmiges Gemisch erhalten wird.

2. Verfahren nach Anspruch 1, **dadurch gekennzeichnet, daß** in Stufe A das gasförmige Reaktionsgemisch, das Acrylsäure enthält, auf eine Temperatur von 120 bis 180 °C abgekühlt wird.

3. Verfahren nach Anspruch 1 oder 2, **dadurch gekennzeichnet, daß** in Stufe A das gasförmige Reaktionsgemisch, das Acrylsäure enthält, in einem Sprüh-kühler abgekühlt wird.

4. Verfahren nach einem der Ansprüche 1 bis 3, das folgende weitere Stufe B umfaßt:
B: Auftrennung des Acrylsäure enthaltenden, gasförmigen Gemischs, wobei eine Leichtsiederfraktion und ein Sumpfprodukt, das Roh-Acrylsäure enthält, erhalten wird.

5. Verfahren nach Anspruch 4, **dadurch gekennzeichnet, daß** die Auftrennung gemäß Stufe B in einer Destillationskolonne durchgeführt wird und die Roh-Acrylsäure im Sumpf der Destillationskolonne erhalten wird.

6. Verfahren nach Anspruch 4 oder 5, **dadurch gekennzeichnet, daß** die im Sumpf der Stufe B anfallende Roh-Acrylsäure teilweise in die Abkühlung gemäß Stufe A zurückgeführt wird.

7. Verfahren nach einem der Ansprüche 4 bis 6, **dadurch gekennzeichnet, daß** die in Stufe B erhaltene Roh-Acrylsäure in Rein-Acrylsäure überführt wird.

8. Verfahren zur Herstellung eines Acrylsäureesters oder eines Gemischs aus zwei oder mehr davon, **dadurch gekennzeichnet, daß** es eine Stufe A, wie in einem der Ansprüche 1 bis 3 definiert, eine Stufe B, wie in einem der Ansprüche 4 bis 6 definiert, sowie eine weitere Stufe C umfaßt:
C: Veresterung der in Stufe B erhaltenen Roh-Acrylsäure mittels einem oder mehreren Alkanoten, wobei ein Veresterungsgemisch umfassend einen oder mehrere Acrylsäureester, einen oder mehrere Essigsäureester, einen oder mehrere Maleinsäureester und oligomere und polymere Acrylsäureester erhalten wird.

9. Verfahren nach Anspruch 8, **dadurch gekennzeichnet, daß** es eine weitere Stufe D umfaßt:
D: Auftrennung des Veresterungsgemisches unter Erhalt eines oder mehrerer Acrylsäureester, sowie eines Auftrennungsgemisches, das einen oder mehrere Essigsäureester umfaßt, und eines Sumpfes, der einen oder mehrere Maleinsäureester und oligomere und polymere Acrylsäureester enthält.

10. Verfahren nach Anspruch 9, **dadurch gekennzeichnet, daß** es eine zusätzliche Stufe E umfaßt:
E: Verseifung des Auftrennungsgemisches, wobei ein erstes Verseifungsgemisch, das ein oder mehrere Alkanole und Essigsäuresalze enthält, oder
Verseifung des Sumpfes, wobei ein zweites Verseifungsgemisch, das ein oder mehrere Alkanole und Maleinsäuresalze, Acrylsäuresalze, Hydroxypropionsäuresalze und polymere Acrylsäuresalze enthält, oder
Verseifung des Auftrennungsgemisches und des Sumpfes, wobei eine Mischung aus erstem Verseifungsgemisch und zweitem Verseifüngsgemisch erhalten wird.

## Claims

1. A process for preparing acrylic acid comprising the following stage A:
A: cooling a gaseous reaction mixture which comprises acrylic acid and is obtained in the gas-phase oxidation to prepare acrylic acid, using an acrylic acid containing not more than 5% by weight of water, to a temperature of from 100 to 190°C, to give a gaseous mixture comprising acrylic acid.

2. A process as claimed in claim 1, wherein the gaseous reaction mixture comprising acrylic acid is cooled in stage A to a temperature of from 120 to 180°C.

3. A process as claimed in claim 1 or 2, wherein the gaseous reaction mixture comprising acrylic acid is cooled in stage A in a spray cooler.

4. A process as claimed in any one of claims 1 to 3, which comprises the following further stage B:
B: separating the gaseous mixture comprising acrylic acid to give a low-boiling fraction and a bottom product which comprises crude acrylic acid.

5. A process as claimed in claim 4, wherein the separation of stage B is carried out in a distillation column and the crude acrylic acid is obtained in the bottom of the distillation column.

6. A process as claimed in claims 4 or 5, wherein some of the crude acrylic acid obtained in the bottom product of stage B is passed back to the cooling stage of stage A.

7. A process as claimed in any one of claims 4 to 6, wherein the crude acrylic acid obtained in stage B is converted into pure acrylic acid.

8. A process for preparing an acrylate, or a mixture of two or more thereof, which comprises a stage A as defined in any one of claims 1 to 3, a stage B as defined in any one of claims 4 to 6, and a further stage C:
C: esterifying the crude acrylic acid obtained in stage B by means of one or more alkanols, to give an esterification mixture comprising one or more acrylates, one or more acetic esters, one or more maleic esters, and oligomeric and polymeric acrylates.

9. A process as claimed in claim 8, which comprises a further stage D:
D: separating the esterification mixture, to give one or more acrylates, and a separation mixture which comprises one or more acetic esters, and a bottom product which comprises one or more maleic esters and oligomeric and polymeric acrylates.

10. A process as claimed in claim 9, which comprises an additional stage E:
E: hydrolyzing the separation mixture, to give a first hydrolysis mixture which comprises one or more alkanols and acetic salts, or
hydrolyzing the bottom product, to give a second hydrolysis mixture which comprises one or more alkanols and maleic salts, acrylic salts, hydroxypropionic salts and polymeric acrylic salts, or
hydrolyzing the separation mixture and the bottom product, to give a mixture of the first hydrolysis mixture and second hydrolysis mixture.

## Revendications

1. Procédé de préparation d'acide acrylique, comprenant l'étape A suivante:
A: Refroidissement d'un mélange réactionnel gazeux provenant de l'oxydation en phase gazeuse pour la préparation d'acide acrylique, qui contient de l'acide acrylique, au moyen d'un acide acrylique contenant au maximum 5% en poids d'eau, à une température de 100 à 190°C, avec obtention d'un mélange gazeux contenant de l'acide acrylique.

2. Procédé selon la revendication 1, **caractérisé en ce que**, dans l'étape A, le mélange réactionnel gazeux, qui contient de l'acide acrylique, est refroidi à une température de 120 à 180°C.

3. Procédé selon la revendication 1 ou 2, **caractérisé en ce que**, dans l'étape A, le mélange réactionnel gazeux, qui contient de l'acide acrylique, est refroidi dans un refroidisseur à pulvérisation.

4. Procédé selon l'une des revendications 1 à 3, comprenant l'étape subséquente B ci-après:
B: Séparation du mélange gazeux contenant l'acide acrylique, avec obtention d'une fraction légère et d'un produit de fond de colonne, contenant de l'acide acrylique brut.

5. Procédé selon la revendication 4, **caractérisé en ce que** la séparation selon l'étape B est entreprise dans une colonne de distillation et que l'acide acrylique brut est recueilli dans le fond de la colonne de distillation.

6. Procédé selon la revendication 4 ou 5, **caractérisé en ce que** l'acide acrylique brut recueilli en fraction de queue dans l'étape B est partiellement recyclé dans le refroidissement selon l'étape A.

7. Procédé selon l'une des revendications 4 à 6, **caractérisé en ce que** l'acide acrylique brut obtenu à l'étape B est converti en acide acrylique pur.

8. Procédé de préparation d'un ester d'acide acrylique ou d'un mélange de deux ou plus de deux d'entre eux, **caractérisé en ce qu'**il comporte une étape A, telle que définie dans l'une des revendications 1 à 3, une étape B, telle que définie dans l'une des revendications 4 à 6, ainsi qu'une autre étape C:
C: Estérification de l'acide acrylique brut obtenu à l'étape B au moyen d'un ou plusieurs alcanols, avec obtention d'un mélange d'estérification comprenant un ou plusieurs esters d'acide acrylique, un ou plusieurs esters d'acide acétique, un ou plusieurs esters d'acide maléique ainsi que des oligomères et des polymères d'esters d'acide acrylique.

9. Procédé selon la revendication 8, **caractérisé en ce qu'**il comporte une autre étape D:
D: Séparation du mélange d'estérification avec obtention d'un ou plusieurs esters d'acide acrylique, ainsi que d'un mélange de séparation contenant un ou plusieurs esters d'acide acétique, et d'un produit de fond de colonne contenant un ou plusieurs esters d'acide maléique ainsi que des oligomères et des polymères d'esters d'acide acrylique.

10. Procédé selon la revendication 9, **caractérisé en ce qu'**il contient une étape additionnelle E:
E: Saponification du mélange de séparation, avec obtention d'un premier mélange de saponification, qui contient un ou plusieurs alcanols et des sels d'acide acétique, ou
Saponification du produit de fond de colonne, avec obtention d'un deuxième mélange de saponification, qui contient un ou plusieurs alcanols et des sels d'acide maléique, des sels d'acide acrylique, des sels d'acide hydroxypropionique et des sels d'acide acrylique polymères, ou
Saponification du mélange de séparation et du produit de fond de colonne, avec obtention d'un mélange constitué du premier mélange de saponification et du deuxième mélange de saponification.
